# EUROPEAN PATENT SPECIFICATION

(11) **EP 1 436 445 B1**
(45) Date of publication and mention of the grant of the patent: **17.05.2006**
(21) Application number: 02758575.1
(22) Date of filing: 30.08.2002
(51) Int. Cl.: C23C 18/12, B01J 35/06, B01J 37/08, B01J 21/06, B01J 37/02

(54) **PROCESS FOR MAKING THIN FILM POROUS CERAMIC-METAL COMPOSITES AND COMPOSITES OBTAINED BY THIS PROCESS**
VERFAHREN ZUR HERSTELLUNG PORÖSER KERAMIK-METALL VERBUNDWERKSTOFFE UND DADURCH ERHALTENE VERBUNDWERKSTOFFE
PROCEDE POUR PRODUIRE DES COMPOSITES EN CERAMIQUE-METAL EN FILMS MINCES POREUX ET COMPOSITES OBTENUS PAR CE PROCEDE

(30) Priority: 30.08.2001 GB 0120958; 30.08.2001 GB 0120961; 30.08.2001 GB 0120956
(43) Date of publication of application: 14.07.2004
(73) Proprietor: Aktina Limited, Lasswade, Central Scotland EH18 1AW (GB)
(72) Inventor: CAIRNS, James, Anthony, University of Dundee, Dundee, Central Scotland DD1 4HN (GB); BERRY, Graham, James, University of Dundee, Dundee, Central Scotland DD1 4HN (GB); CALLON, Gary, John, University of Dundee, Dundee, Central Scotland DD1 4HN (GB); SMITH, Robert, Dermot, University of Dundee, Dundee, Central Scotland DD1 4HN (GB)
(86) International application number: PCT/GB2002/004086
(87) International publication number: WO 2003/021004

(56) References cited:
- US-A- 4 464 482
- US-A- 5 698 267
- DEKI S ET AL: "PREPARATION AND CHARACTERIZATION OF AU-DISPERSED TIO2 THIN FILMS BY A LIQUID-PHASE DEPOSITION METHOD" JOURNAL OF MATERIALS CHEMISTRY, THE ROYAL SOCIETY OF CHEMISTRY, CAMBRIDGE, GB, vol. 6, no. 12, 1 December 1996 (1996-12-01), pages 1879-1882, XP000690489 ISSN: 0959-9428
- TANAKA ET AL.: "Preparation of zirconia and zirconia-yttria coatings from metal alkoxide solutions" CHEMICAL ABSTRACTS + INDEXES, AMERICAN CHEMICAL SOCIETY. COLUMBUS, US, vol. 115, 16 December 1991 (1991-12-16), XP000318737 ISSN: 0009-2258

## Description

### TECHNICAL FIELD

The present invention relates to the manufacture of thin film porous ceramic-metal composites comprising a support substrate with a layer of porous ceramic having metals or metal oxides dispersed therein. More particularly, the invention relates to the manufacture of composites comprising a layer of porous stabilized zirconia in a suitable stable crystalline modification such as the cubic phase, incorporating metal, such as palladium or platinum, in highly dispersed form. The invention is particularly applicable for use as a catalyst for the combustion of hydrocarbons and as gas sensors, for example as a CO sensor.

### BACKGROUND OF THE INVENTION

With the advent of modem antipollution laws in Europe and around the world, significant and new methods of minimizing various pollutants are being investigated. The burning of fuel, be the fuel wood, coal, oil, or a natural gas, likely causes a majority of the pollution problems in existence today. Pollutants, such as CO, are created as a result of imperfect combustion and may be removed by treating the exhaust gas produced. Most of these processes utilize catalysts containing metal or metal oxide on ceramic or metallic substrates. Platinum group metals are known to be useful in catalytic combustion processes.

The pollution problem also demands the development of new highly sensitive gas sensors, capable of providing detection of the pollutants at lower levels than before, due to more severe requirements pertaining to the quality of purification of effluent gases and lower concentration limits applicable to vehicle catalysts.

For this reason, much of the development work is directed at catalysts, composites and gas sensors capable of withstanding high temperatures and yet remaining active and highly durable, and at gas sensors having high sensitivity at low concentration of contaminants.

Commonly, catalytic material and gas sensors are provided on substrates, such as metallic or ceramic substrates. The substrates need to have very open structures in order to allow gas mixtures to interact with them efficiently. For instance, wire meshes, metallic or ceramic bodies provided with holes, and boards or meshes made of metallic or ceramic fibers have all been used.

To further increase the boundary surface of heterogeneous catalysts, composite materials are widely used, which comprise a substrate as mentioned above coated with a porous ceramic layer incorporating or having applied thereon a catalytic metal or metals.

Conventionally, catalytic material is applied to substrates by immersing the substrate in a washcoat slurry, which may also contain the catalytic material. Lately, organic solutions of metal organic precursors of catalytic material have become widely used instead of slurries.

Thus, according to US 6,303,538, a catalyst is prepared by pre-oxidising a substrate, preparing a solution comprising a ceramic oxide precursor, such as alumina or silica, and a solution containing a catalyst precursor, depositing precursors onto the substrate, such as a fiber board, and thermally treating the board to decompose the precursor into the catalyst. The precursors are deposited by immersing, spraying or the like methods.

The metallic fiber board obtained thereby comprises a plurality of metallic fibers, preferably produced from a Fe-Cr-Al alloy, wherein the fibers are coated with a dense oxide covering the surfaces of the fibers, onto which a porous oxide layer is deposited covering the exterior surface of the dense layer, and the catalyst is a noble metal distributed across the exterior surface of the porous oxide layer or incorporated within it.

US 5,980,843 discloses another catalytic system obtained from a perforated foil or a metallic grid on which a porous ceramic layer, preferably of alumina or zirconia, is deposited by means of techniques including plasma spraying, flame spraying, and detonation spraying. The ceramic layer is then impregnated with a catalyst precursor solution or suspension. After suitable thermal treatments the final catalytic system is obtained.

The production processes described above, however, are disadvantageous in that, predominantly, the ceramic oxide layer formed from precursor solutions has an amorphous structure, and hence, low structural strength, insufficient thermal stability and low adhesion to the substrate.

Several approaches are known to transform amorphous structures of ceramic layers obtained from organic precursors.

Thus, according to US 6,027,826, a porous amorphous metal oxide ceramic deposit is formed directly on the substrate by spray pyrolyzing a solution of metalorganic precursors mixed at the molecular level onto the metallic substrate, which is previously heated to temperatures greater than the boiling point of the organic solvent and which are high enough to initiate in situ decomposition of the metalorganic precursor salts. The resultant porous amorphous oxide deposit is processed into a fully crystallized c-axis oriented ceramic through a series of mechanical compression and short reaction treatments that are used to crystallize the amorphous ceramic layer.

Another approach to post-deposit treatment is described in WO 00/22664, according to which an amorphous metal oxide film is deposited over a substrate using a metal organic precursor, the substrate is then heated in an inert ambient at a temperature greater than the crystallization temperature to convert the amorphous metal oxide to a polycrystalline metal oxide which is then heated in an oxygen containing ambient.

Still one more approach is described in US 5,786,294 according to which the substrate is treated chemically; in particular, sulfate deposition is preferred for the transformation of a mesoporous precursor with amorphous pore walls into a material with crystalline pore walls maintaining the mesoporous characteristics. A catalyst material is obtained thereby, comprising zirconium dioxide particles with a mesoporous matrix, said mesopores having walls with a substantially tetragonal crystalline structure; and a stabilizing chemical moiety on the surface of said mesoporous matrix.

According to US 5,698,267, a porous ceramic layer of yttria-stabilised zirconia is applied on a cermet electrode as a paste containing high percentage of these oxides. The method involves high temperature (1300-1600°C) sintering and results in a composite comprising a relatively thick layer of zirconia on cermete electrode.

Thus, all the above discussed methods require the use of high temperature processing of the ceramic substrate or the use of expensive equipment, such as plasma spraying.

Moreover, the known approaches, though effective in obtaining crystalline ceramic structures and overcoming drawbacks of amorphous ceramic substrates, still do not offer a suitable solution to another problem, which is low adhesion of catalytic layers to metal substrates, and hence, low structural integrity of the catalytic members itself.

### SUMMARY OF THE INVENTION

It is an objective of the present invention to obviate and/or mitigate at least one of the aforementioned drawbacks.

It is another objective of the invention to provide a relatively simple and cost-effective method of preparing porous ceramic-metal composites having high temperature stability and structural integrity.

Still another objective is to provide a simple and convenient method of coating a substrate having low adhesion properties with a porous ceramic material incorporating one or more metals.

Still one more objective is to provide a catalytic member for combustion of gases, having a high thermal conductivity and low flow resistivity but at the same time improved mass transfer characteristics and high catalytic activity.

Still another objective is to provide a sensor element for a gas sensor having high sensitivity at low concentration of gases and useful reaction kinetics.

Still another objective is to provide a convenient method of depositing metal, including patterned metal, on to various substrates, including, but not limited to, glass and polymers, and to achieve good adhesion between the metal and the substrate.

These and other objectives are surprisingly achieved by the process of the present application which teaches how to prepare thin film stabilised porous ceramic, incorporating a suitable oxide, such as zirconia in a suitable crystalline modification, such as the cubic phase, which can be effectively used in making catalysts and gas sensors and such that avoid or eliminate a decrease in catalyst/sensor activity at high temperatures and provide thermally and mechanically stable composite structures.

By using highly stabilised porous ceramic/metal structures of the present invention, problems associated with the structural integrity of the composite member, such as loss of surface area of the ceramic, and sintering of the catalytic metal can be minimized or eliminated. These advantages also apply to gas sensors.

The inventive approach of the present application as set forth in the claims is to introduce the stabilising oxide into the precursor solution, so that the transformation of the amorphous porous ceramic layer into a crystalline structure surprisingly occurs at lower temperatures and relatively milder conditions than usually employed to effect such a transformation. The result is that the porous oxide layer is bonded strongly to the substrate and has a high structural strength, avoiding the necessity of providing additional treatment of the composite material.

According to the first aspect of the present invention, a process for the production of a thin porous film, composed of a suitable material, such as zirconia in a suitable crystalline form such as the cubic phase is provided, comprising the steps of:
- providing a substrate;
- providing a first solution comprising an organic precursor compound of a zirconia matrix material in an appropriate organic solvent;
- providing a second solution comprising an organic precursor compound of a stabilising oxide for the ceramic matrix in an appropriate organic solvent
- providing a third solution of an organic compound of one or more metals;
- contacting the solutions with the substrate so as to form a coating comprising the zirconia precursor, the stabilising chemical moiety precursor, and the metal precursor;
- thermally treating said coated substrate at a temperature sufficient to decompose said precursor compounds to form the substrate having thereon a thin film of stabilised zirconia having a suitable crystalline form such as the cubic phase incorporating therein or thereon said one or more metals.

The solution of the stabilising oxide is prepared in a suitable solvent separately or it may be dissolved in the first solution, or both compounds can be dissolved in one solvent. Alternatively, the stabilising oxide can be prepared in a solvent which differs from the solvent used to prepare the first solution. Similarly, the metal organic compound can be dissolved in the same solution to make a composite in which the metal is incorporated in the porous ceramic layer, or a separate solution can be prepared to apply the metal precursor onto the porous ceramic layer.

The substrate can be contacted sequentially with the first solution, then, with the second solution, and, then, with the third solution. However, preferably, the first and the second solution are mixed, so as to obtain the stabilised porous ceramic precursor solution, and the mixture is applied onto the substrate. After contacting the substrate with the mixture of the first and second solutions, it may be necessary to dry or consolidate the first layer of coating before depositing a metal organic compound. It is also possible to mix the first, second and third solutions, using a common solvent.

Consolidating is understood to mean heating the coated substrate for a period of time and at an appropriate temperature. During this consolidating step the matrix material may be converted to a stable phase, for example if the matrix material comprises zirconia the stable phase may be cubic zirconia.

The precursor solutions can be deposited onto the substrate by immersing the substrate into the precursor solutions, or atomizing said precursor solutions and directing a spray onto said substrate and the like. One of the great advantages of the present approach is that it enables the coating to be applied readily to a whole range of substrates. In the case of a flat surface, this may be done by, for example, spin coating, whereas in the case of substrates of an irregular shape, such as fibres or wires, it may be done simple by immersing the substrate in the solution, followed by drying and subsequent heat treatment. Coatings may also be applied by other means, for example by electrostatic spraying or airless ultrasonic spraying. The solution, being based on an organic solvent, produces a uniform coating on a wide range of substrates, including silicon, metals such as steel, polymers such as polyimide, and glass. After drying and thermal curing, it exhibits excellent adhesion.

In one preferred embodiment, the substrate comprises metal, such as steel, or alloys, such as containing iron, chromium and aluminum, for example, Fecralloy steel, US 4,330,476 (Cairns et al).

In one more preferred embodiment, the substrate comprises a knitted wire monolith, such as in the form of a sheet-like material or a roll, for example as described in US 4,397,772 (Cairns et al), or US 4,464,482 (Bird et al).

Alternatively, the substrate may be formed of silicon, polymers, such as polyimide, ceramics and glass.

The organic precursor or precursors of the ceramic layer may be any suitable organic compounds which may be converted to a porous ceramic material on heating. It is understood that the porous ceramic material is porous to gases and/or liquids. Examples of suitable organic compounds include aluminium or zirconium substituted or unsubstituted C₁-C₈ alkyl carboxylates, such as propionate or ethylhexanoate.

The organic precursor is dissolved in an appropriate organic solvent. This will be determined by the nature of the organic precursor and may for example be ethanol, methanol, dimethylsulfoxide, tetrahydrofuran, chloroform, hexane, dichloromethane, ethylacetate, acetone, diethylether and the like. An organic precursor comprising compounds of zirconium and yttrium may for example be dissolved in tetrahydrofuran.

Preferably, the ceramic precursor is an organic compound of zirconium, such as zirconium substituted or unsubstituted C₁-C₈ alkyl carboxylate, such as zirconium propionate.

The method allows the ceramic materials, such as zirconia, to be stabilised by the addition of stabilising oxide precursors which may be any suitable organic compound of yttrium or cerium, such as yttrium substituted or unsubstituted C₁-C₈ alkyl carboxylate. According to the invention, the stabilisation is performed under moderate conditions in the process of structuring the composite material, so asto convert the porous ceramic material into a preferred stable crystalline phase, contrary to the prior art methods employing additional steps to crystallize originally amorphous ceramic material. For example, yttria-stabilised zirconia may be prepared by using zirconium propionate and yttrium 2-ethylhexanoate.

The ceramic precursor may be also an organic compound of aluminium, such as aluminium 2-ethylhexanoate, while cerium 2-ethylhexanoate can be the organic precursor of the stabilising moiety.

In practice the yttria and/or ceria generally forms a minor component of the stabilised porous ceramic composite and as such typically comprises less than 20%, preferable less than 10% by weight of the composite material. In some cases however the ceria is the predominant component, comprising up to 70% by weight of the composite material.

An organic compound of the metal to be incorporated in the matrix material may also be dissolved in the solution. Typical metals include palladium, platinum, rhodium, copper, silver, nickel, gold and the like, as well as mixtures thereof, and convenient compounds include the acetate, acetyl acetonate, alkyl halide, etc. The amount of metal as compared to the matrix material will depend on the ultimate application. Generally speaking, however, the amount of the metal as compared to the matrix material may be between 5% to 80% by weight more preferable 10% to 60%, such as about 15%, 20%, 30%, 40% or 50%.

The thin film stabilised porous substantially crystalline ceramic is formed on said substrate by thermal oxidation carried out by heating said substrate in an atmosphere selected from the group of air, oxygen, nitrogen, mixtures of air and oxygen, mixtures of oxygen and nitrogen, water vapor, and combinations thereof.

The heating is carried out at a temperature in the range from about 350°C to about 1000°C, typically 400°C, for a period between about 10 seconds to about 10 minutes.

The heating may be also carried out at a temperature in the range from about 400°C to about 1000°C for a period in the range from about 10 minutes to about 2 hours.

It should be appreciated that though the effect of stabilising zirconia by yttria is well known, typically, to achieve this, it is required that zirconia is treated at high temperatures to ensure that the two oxides are mixed to the extent that a solid solution is formed and free ionic vacancies in the zirconia crystalline lattice are created by the incorporation of yttrium atoms. This is especially the case when powders of zirconia and yttria are used. However, according to the method of the present invention, effective stabilisation is achieved under significantly more moderate conditions due to the use of organic precursors as described in detail hereinbelow and illustrated by non-limiting examples.

According to the second aspect of the invention, a thin film porous ceramic-metal composite is provided, comprising:
- a substrate;
- a porous ceramic layer of substantially crystalline structure stabilised by a stabilising oxide, wherein the ceramic layer is adhered directly to the substrate by strong bonding of the metal oxide of the ceramic to the substrate, and
- a metal incorporated in or on the ceramic substantially crystalline layer.

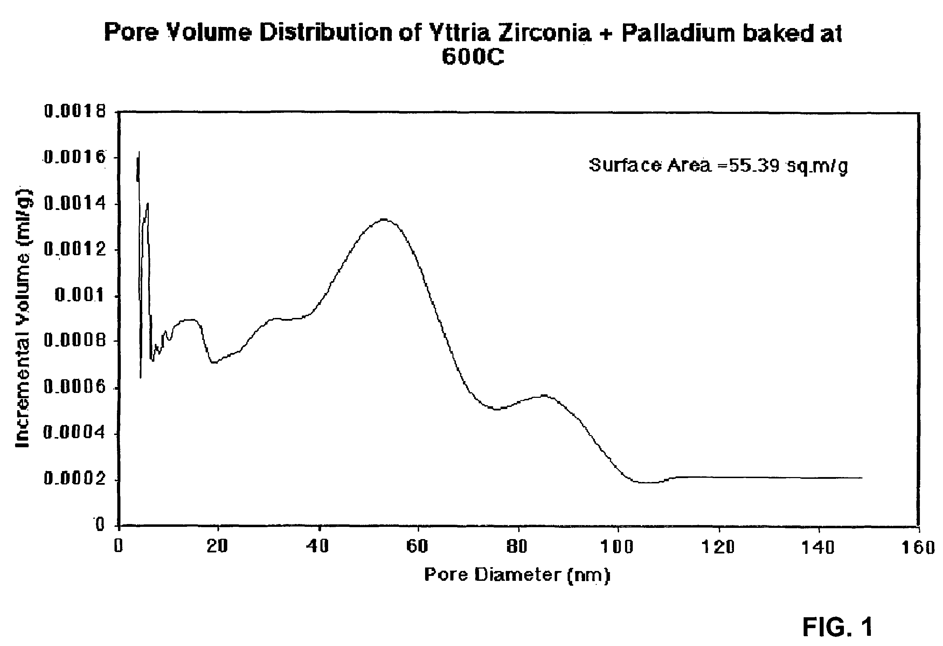

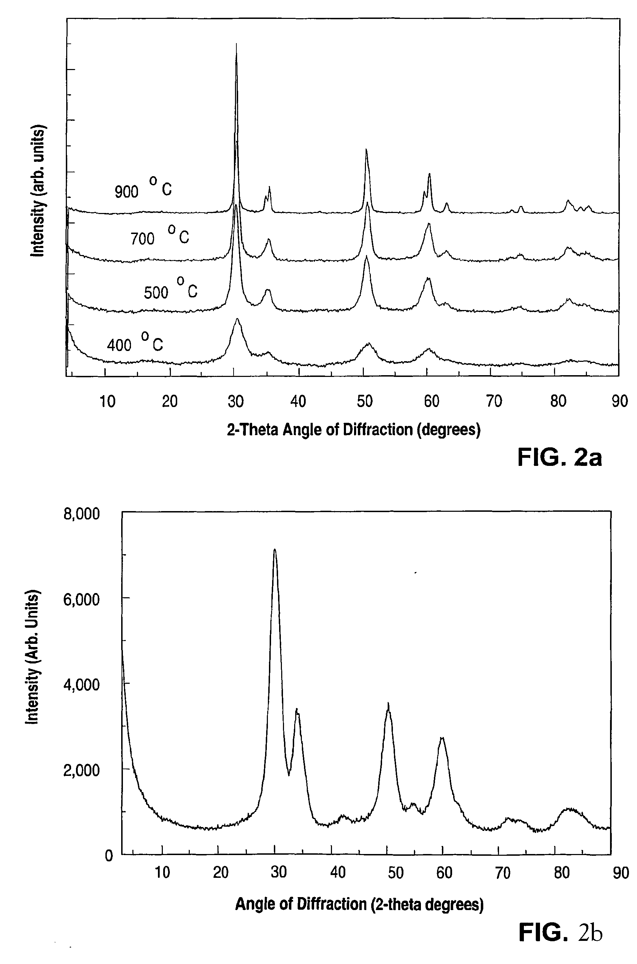

The crystalline nature of the ceramic layer has been confirmed by using X-ray diffraction as shown in Figs. 2a and 2b.The porous properties of the ceramic layer have been confirmed by gas adsorption techniques to measure surface area and pore volume distribution methods, as shown in Fig.1 and by the following examples.

Preferably, the thin film catalytic member according to the invention is made on a substrate which is a metal wire, such as a knitted wire material or knitted wire material which is rolled to form a cylindrical member.

The wire diameter is preferably from 0,12 mm to 0,25 mm.

The porous ceramic layer is preferably an yttria-stabilised zirconia in a preferred crystalline form such as the cubic phase.

According to another example embodiment, the porous ceramic layer is an alumina stabilised by ceria.

Preferably, the thickness of the porous ceramic layer is from 1 nm to 10 µm.

In another preferable embodiment, the thickness is from 50 nm to 500 nm, in still another preferable embodiment, less than 80 nm.

The metal is a catalytic metal, for example taken from the 8^{th} group of the Periodic Table, such as mentioned above. In one of the preferred embodiments, the catalytic metal is palladium in a concentration from about 0,5% by weight to about 15% by weight to the weight of the ceramic material

The thin film composite may further comprise rhodium in a concentration from 0,05% to 5% by weight to the weight of ceramic material.

The composite exerts catalytic capability with respect to exhaust vehicle gases, including combustion of hydrocarbons and carbon monoxide and reduction of NOx. Also, the composite may be used to deal with other combustible gases, such as propane, butane and the like.

In the third aspect, the present invention provides a metallic wire catalyst member comprising a metallic wire support material formed of a knitted metallic wire or wires, wherein the wire (or wires) is coated with a thin film porous oxide ceramic material having a stabilised substantially crystalline structure, the ceramic incorporating thereon or therein one or more catalytic metals.

The new type of vehicle exhaust catalyst prepared using the thin films of the present invention is much less likely to suffer from drawbacks of the prior art and offers several advantages over conventional vehicle exhaust catalysts, including the following ones.

First, they can be applied as much thinner coatings than technology using water or aqueous alcoholic mixtures, such as described in US 6,303,538. In the thick coatings produced by the use of such aqueous slurries some of the catalytic metal is likely to be buried in the coating, and therefore is not used effectively. The result is that the amount of precious metal used in the catalysts produced by the thin film technology as described in the present invention can be reduced substantially, by as much as a factor of five.

Second, the thin films can be applied readily to a whole range of substrates, including metal wire, to which they exhibit excellent adhesion. Coatings derived from aqueous slurries exhibit poor adhesion to such metal substrates, and are likely to fall off in use, causing a serious loss of valuable precious metal. Thus, according to US 3,606,538, a separate preoxidation step is required to form a dense oxide layer in an attempt to enhance thereby the surface adherence of the coating to the metal substrate. The catalysts of the present invention are found to exhibit excellent adhesion to the steel wire. Typical wire diameters range from 0.12 mm to 0.25 mm, but other sizes outwith this range may be used depending on application.It should be noted also that the use of metal wire substrates offers additional significant advantages. For example, they confer improved mechanical durability, are easily installed into the exhaust system, and also prevent the generation of localised hot spots, which may cause the catalyst in such regions to sinter and lose surface area. The wire substrates also offer improved catalytic performance. This arises because conventional ceramic monoliths contain a multitude of flow channels of high aspect ratio (i.e. ratio of channel length to channel cross-sectional area). This causes the gas to develop a boundary layer along the length of the channel (see U.S. Patent Number 5,440,872) which results in the catalyst being mass-transfer limited, thereby reducing the efficiency of the interaction of the gas with the catalyst surface.

A third advantage of the thin film technology is that it enables the catalyst support material to be synthesised in its most stable crystalline phase during the preparation of the catalyst. Vehicle exhaust catalysts must be able to operate at temperatures of 1000°C and above, and therefore the inorganic support material on which the catalytic metal is deposited should be in its most stable crystalline form. As a case in point, it is well known that zirconium oxide can be converted into a stable crystalline phase by being doped with oxides of certain elements, such as yttrium. This normally requires processing at elevated temperatures: as high as 1200°C in the case of powders. However in the thin film route described here it has been found that the stabilised phase of zirconium oxide can be produced in-situ, and starts to form at temperatures as low as 350°C. Thus this route can be used as a general method of preparing catalysts, including those containing several metals and several inorganic components, all of which can be brought together as solutions of organic precursor salts. This ensures efficient interaction between the components and enables the catalyst to be synthesised in its desired crystalline phase during its synthesis. This is in contrast to conventional catalyst preparation methods which use support materials which have been synthesised separately (often at high temperatures).

Thus, in a preferred embodiment the present invention provides a method of preparing a thin film porous ceramic-metal composite having catalytic functionality, comprising:
- providing a metallic wire material formed of a knitted metallic wire or wires;
- providing a first solution comprising an organic precursor of zirconia and an organic precursor of a stabilising oxide for zirconia in an appropriate organic solvent to form a stabilised precursor solution;
- providing a second solution of organic compound of one or more catalytic metals;
- forming a coating adhered directly to the substrate by contacting the metallic wire substrate with said solution or solutions;
- thermally treating said substrate at a temperature sufficient to decompose said precursor compounds to form a porous stabilised ceramic material of substantially crystalline structure incorporating said one or more catalytic metals.

Preferably, the knitted wire material is made of Fecralloy steel as mentioned above. Also preferably, the metallic knitted wire catalyst material is rolled up to form a cylindrical member, but other geometric shapes can be used. The substrate of knitted wire material provides a very open structure that allows gases to pass easily.

According to the invention, the metallic knitted wire catalyst member comprises a metallic wire (or wires) knitted material, wherein the wire (or wires) is coated with a porous stabilised ceramic material of substantially crystalline structure, incorporating one or more catalyst metal or metals, wherein the ceramic material is strongly adhered to the substrate.

In an attempt to provide a good adherence of the ceramic matrix to a metallic substrate, a traditional approach has been, as mentioned above, to form an oxidised sub-layer on the surface of the metal substrate. It was believed also (see US 4,397,772 to Noakes and Cairns, and US 6,303,538 to Toia) that a Fecralloy substrate comprising an alloy of iron with additions of chromium, aluminium and yttrium has the property of forming a substantially alumina surface layer on heating in air, which layer is tenaciously adherent to the alloy substrate and thus, especially beneficial for subsequent coating with a porous ceramic matrix. However when the substrate is in the form of a knitted wire, it is difficult to obtain good adhesion to the relatively thick coatings of ceramic prepared by traditional methods.

The inventive approach of the present patent is to introduce the stabilising oxide into the precursor solution, so that the porous oxide layer itself is bonded strongly to the substrate and has a high structural strength, avoiding the necessity of providing an intermediate dense oxide layer.

It has been discovered that such a porous ceramic structure of high strength can be obtained when a ceramic oxide precursor is mixed with a stabilising oxide precursor and the resulting mixture is applied directly to the substrate to form a coating.

The present invention is advantageous for several reasons.

It provides a convenient method of applying to a substrate a porous, stabilised ceramic layer, within which is incorporated one or more metals or metal oxides. The layer exhibits outstanding adhesion to the substrate. Also, since the layer is much thinner than conventional ceramic coatings, the catalytic metal has good access to the reactant gases. This allows the metal content of the catalyst to be reduced significantly. Additionally, the metal particles can serve as nuclei during the subsequent post-treatment steps (if any), for example, when plating a metal onto the ceramic thin film.

In a further aspect, an enhanced sensing element is provided for solid state gas sensors using the process described in the present application.

This is another example of a product which benefits from having its components interact as precursors during its synthesis, because it enables the desired structures to be created with high efficiency. The thin film technology described here allows these sensors to be fabricated as miniature components (typically 2mm long and 1mm wide) from a single film, thereby enabling them to be mass-produced. Since the active layer of the sensor is very thin (of the order of 200 nanometres) and highly porous, the sensor is very sensitive and responsive. As an example of the fabrication of a sensor using the thin film technology described here, organic solutions of the precursors of zirconia, alumina, and palladium, were used to produce a porous inorganic zirconia/alumina matrix, associated with highly dispersed palladium oxide. This intimate interaction between all of these components resulted in a sensor, which was able to respond rapidly to changes in the concentration of gases, such as carbon monoxide.

In a further aspect, the process of the invention is employed to produce a thin film metal coating on low adhesion substrates.

Suppose for example that it is desired to deposit a metal film, such as copper, on to a glass substrate. Conventionally this is done in a vacuum system, with the metal being deposited on to the substrate by evaporation or sputtering. However by using the thin films of the present invention, there is no need to use a vacuum system because the films can simply be coated on to a substrate, then subjected to electroless deposition of copper in an appropriate chemical bath. In this case palladium nuclei within the porous film act as catalytic sites, on to which the copper is deposited. The resulting copper films exhibit excellent adhesion to the substrate, due, it is believed, to the copper nuclei being dispersed throughout the porous film. It is important to note also that, in contrast to the conventional method, the substrate need not be flat. Substrates of any shape, including fibres, can in this way be coated uniformly with metal.

Consider now the situation in -which there is a requirement for the metal, such as copper, to be deposited as a film, in the form of a well-defined pattern on a substrate. In order to achieve this by conventional processing, it would be necessary first to deposit the copper film in a vacuum system as described above, and then to coat the copper film with a layer of resist. The resist layer would then be irradiated with ultra-violet light through an appropriate mask. Having developed the resist layer to produce the desired pattern, the copper thereby exposed would be etched. Finally, the resist would be removed from the remaining copper, to leave the copper pattern.

In contrast, the thin films of the present invention offer a much simpler means of producing metal patterns. In this case a solution containing the precursors of the inorganic matrix (such as zirconia) and the metal (such as palladium) is deposited on to the substrate, and allowed to dry under ambient conditions. The film is then maintained at a temperature below that necessary to cause decomposition of the precursor molecules. When the resulting film is subjected to ultra-violet irradiation through an appropriate mask, the irradiated regions of the film are rendered less soluble than the non-irradiated regions. That is, the film behaves as a negative resist. Therefore when the film is subsequently immersed in an appropriate organic solvent, the non-irradiated regions are washed away. When the remaining film is dried and heated, and subjected to electroless plating, copper can be deposited in the form of the desired pattern.

The strong adhesion of the metal film to the ceramic layer has been confirmed by a Scotch tape test as described in Example 7. These aspects and embodiments of the present invention provide advantages over prior art methods for producing thin film porous stabilised ceramic layers and products obtained thereby. Techniques of the prior art such as using various pre- and post-preparation treatment steps to convert amorphous ceramic to stable crystalline form tend to be cost-ineffective and require relative expensive equipment. The present invention allows for a much more simple and effective process of preparing composite materials which can find application in various fields. Thus, the method provides imparting to ceramic material significantly more catalytic functionality than would otherwise be possible using traditional methods of preparing catalysts. Further, this embodiment allows for the production of catalytic materials with complex geometry. The process of the invention is further advantageous for reducing the total number of processing steps and the total processing time necessary to produce a porous metal-ceramic composite with a stable crystalline structure.

Additional advantages of the present invention will become apparent to those skilled in the art upon reading of the following detailed description of the preferred embodiments, the examples, and the figures.

### BRIEF DESCRIPTION OF THE DRAWINGS

FIG. 1 Pore volume distribution of yttria-stabilised zirconia incorporating palladium prepared according to the invention;
FIG. 2a X-ray diffraction spectrum of 9% yttria-stabilised zirconia sintered at temperatures according to the invention;
FIG. 2b X-ray diffraction spectrum of yttria-stabilised zirconia incorporating 10% of palladium to zirconium sintered at temperatures according to the invention;
FIG. 3 Transmission electron micrograph of platinum particles in an yttria-stabilised zirconia matrix;
FIG. 4 Conversion efficiency of catalyst according to the invention with respect to carbon monoxide gas when subjected to thermal cycling;
FIG. 5 Response of a gas sensor element prepared according to the process of the invention as a function of carbon dioxide concentration in the ambient air;
FIG. 6 Response of a gas sensor element prepared according to the process of the invention as a function of oxygen concentration in the ambient air;
FIG 7. Example of patterned nickel plated onto polyimide substrate according to the process of the invention.

### DETAILED DESCRIPTION OF THE PREFERRED EMBODIMENTS

The present invention will be further illustrated by the following examples. These non-limiting examples illustrate some embodiments and are intended to teach those skilled in the art how to put the present invention into practice.

### EXAMPLE 1

### Characterisation of yttria-stabilised zirconia incorporating palladium

A sample of yttria-stabilised zirconia incorporating palladium was prepared by dissolving the following ingredients, per litre of tetrahydrofuran:
32g zirconium propionate
8g yttrium 2-ethylhexanoate
3.2g palladium acetate.

The solvent was removed by gentle heating and the resultant powder was cured by baking for 4 hours at 600°C.

The porous nature of the matrix was confirmed by gas adsorption measurements. Thus Figure 1 shows a sample of palladium-containing yttria-stabilised zirconia, the latter having an overall surface area of 55.39m²g⁻¹ with a maximum in the distribution curve at around 50nm. As result of an X-ray diffraction analysis, Figs 2a and 2b show peaks associated with a stable zirconia crystalline phase.

### EXAMPLE 2

### Characterisation of yttria-stabilised zirconia incorporating platinum

A sample of yttria-stabilised zirconia incorporating platinum was prepared by dissolving in dichloromethane the following ingredients, per litre of solvent:
32g zirconium propionate
4g yttrium 2-ethylhexanoate
2.9g dichloro (1,5-cyclooctadiene) platinum

A silicon nitride membrane of dimensions 0.5mm □ 0.5mm □ 100nm thickness supported in a 3mm square silicon frame was immersed into the solution and allowed to dry. It was then baked at 900°C for 2 hours and allowed to cool to room temperature.

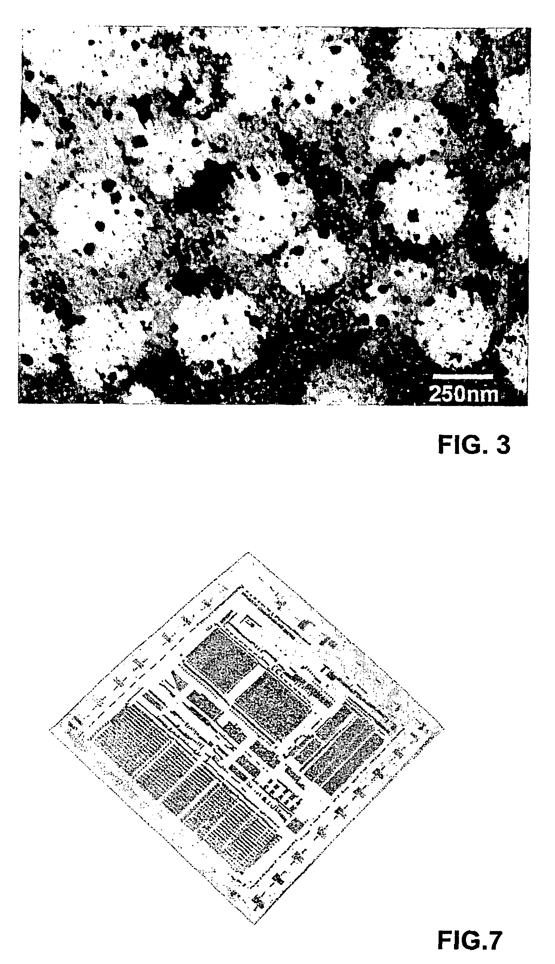

Figure 3 is a transmission electron micrograph of this film, in which the platinum is visible as dark coloured particles.

### EXAMPLE 3

### Preparation of and characterisation of exhaust catalyst

A three way catalyst (i.e. designed to remove carbon monoxide, hydrocarbons and oxides of nitrogen) was prepared from two metal wire substrates, which were interwound with each other. A length of knitted Fecralloy* steel wire of sufficient size to be rolled up to form a cylinder 6 inches (~150mm) in length by 4 inches (~100mm) in diameter, and having a mass of approximately 400g, was cleaned in a hot detergent solution, rinsed and dried. The wire was then cut into two sections, one approximately 10 times longer than the other.
*Fecralloy steel is a patented ferritic steel comprising iron, chromium, aluminium and yttrium.

Part 1. The larger part of the wire was immersed in a solution containing, per litre of tetrahydrofuran, the following ingredients:
20g of palladium acetate
160g of zirconium propionate
50g of cerium 2-ethylhexanoate
17g of aluminium 2-ethylhexanoate
50g of zirconia/ceria/lanthana powder.

It was then removed from the solution, and left to dry for 1 hour at 100°C, followed by rapid heating to 700°C.

Part 2. The smaller part of the wire immersed in a solution containing, per litre of tetrahydrofuran, the following ingredients:
20g of rhodium acetate
160g of zirconium propionate
50g of cerium 2-ethylhexanoate
10g aluminium 2-ethylhexanoate
50g of zirconia/ceria/lanthana powder
and subsequently treated in exactly the same manner as described for part 1 above. After firing, the precious metal (palladium) loading of part 1 was approximately 0.3g, and the rhodium loading of part 2 was approximately 0.05g.

The catalyst monolith was fabricated by rolling the two lengths of wire together to form a cylinder approximately 6 inches (150mm) in length and 4 inches (100mm) in diameter.

The catalyst produced in this manner was tested on an engine mounted on a test bed, and shown to exhibit good performance, as illustrated by the data in Table 1 below. The gas analysis was performed using a Signal Instruments system, comprising a 4000VM NOₓ analyser, a 3000HM THC analyser (hydrocarbons), two 7000FM GFC analysers (CO and CO₂) and an 8000M O₂ analyser.

**Table 1**

| | Pre catalyst | Post catalyst | Conversion |
|---|---|---|---|
| NOₓ (ppm) | 2981 | 737 | 75% |
| Total hydrocarbon (ppm) | 327 | 15 | 95% |
| Carbon monoxide (%) | 0.43 | 0.053 | 88% |
| Oxygen (%) | 0.121 | 0.057 | |

### Table 1- Engine test data

The engine used for this test was a 1.81 8 valve normally-aspirated VW GTi unit operated at 2500rpm and under a load of 100Nm. The space velocity of the exhaust gas over the catalyst was approximately 70000 (normalised to STP).

### EXAMPLE 4

Preparation of exhaust catalyst using a stainless steel substrate.

A three way catalyst (i.e. designed to remove carbon monoxide, hydrocarbons and oxides of nitrogen) was prepared from a metal wire substrate. A length of knitted 310 stainless steel wire of diameter 0.15mm and of sufficient size to be rolled up to form a cylinder 6 inches (~150mm) in length by 4 inches (~100mm) in diameter, and having a mass of approximately 400g, was cleaned in a hot detergent solution, rinsed and dried.

The wire was immersed in a solution containing, per litre of tetrahydrofuran, the following ingredients:
20g of palladium acetate
20g of rhodium acetate
160g of zirconium propionate
   50g of cerium 2-ethylhexanoate
   67g of aluminium 2-ethylhexanoate
   50g of titania powder
   50g of manganese (III) acetate.

It was then removed from the solution, and left to dry for 1 hour at 100°C, followed by rapid heating to 700°C.

After firing, the precious metal (palladium) loading of the monolith was approximately 0.3g, and the rhodium loading was approximately 0.05g.

The catalyst monolith was fabricated by rolling the length of wire to form a cylinder approximately 6 inches (150mm) in length and 4 inches (100mm) in diameter.

The catalyst produced in this manner was tested on the same engine, under identical load conditions, and analysed using the same equipment as in the previous example.

Table 2 below shows engine test data.

| | Pre catalyst | Post catalyst | Conversion |
|---|---|---|---|
| NOₓ (ppm) | 3209 | 397 | 81 % |
| Total hydrocarbon (ppm) | 397 | 23.6 | 94% |
| Carbon monoxide (%) | 0.47 | 0.0686 | 85% |
| Oxygen (%) | 0.345 | 0.00 | |

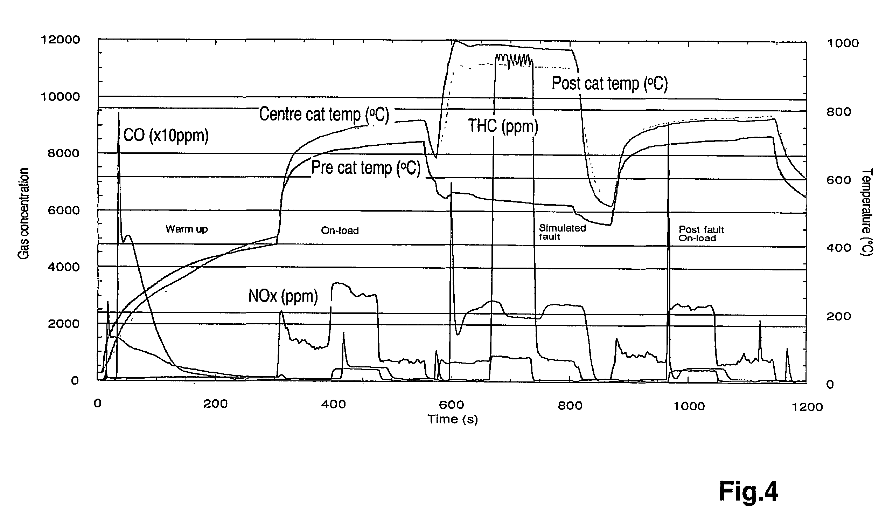

Part of the engine test involved the removal of a spark plug lead from the engine to simulate a fault which may occur on a real car. Under such circumstances a large quantity of unburned fuel reaches the catalyst and is combusted thereon, raising the monolith to a very high temperature, typically 1000°C. This provides a severe test for any catalyst, but it can be seen from Figure 4 that after the fault was rectified the conversion efficiencies of the catalyst recovered to their previous values, indicating that no damage had been sustained by the high temperature excursion. This is an important factor in ensuring the longevity of a vehicle exhaust catalyst, which will typically be expected to last for 100,000 miles (160, 000km).

To explain in more detail, Figure 4 shows the temperature of the exhaust gases measured at three points: immediately before the catalyst (pre cat); in the centre of the wire monolith (centre cat); and immediately downstream of the catalyst (post cat). The three gas concentrations shown are oxides of nitrogen (NOx), total hydrocarbons (THC) and carbon monoxide (CO). Note that this last trace has been expanded by a factor of 10 for clarity.

The engine test comprises a 300s warm up with the engine idling off-load, then a load is applied and the engine speed increased. The exhaust gas is sampled pre- and post-catalyst, in order to facilitate the calculation of conversion efficiencies of the various exhaust gas components. This accounts for the large step changes seen in the traces. The load is then removed and a fault condition is simulated, as noted above, with the engine running at approximately 2500rpm. Again pre- and post-catalyst measurements are taken. The fault is then rectified and the load applied once more, and again pre- and post-catalyst measurements are taken. Finally the load is removed and the engine is switched off.

### EXAMPLE 5

### Preparation of resistive sensor for combustible gas detection

A piece of alumina tile having dimensions 10mm by 20mm by 1mm thick was coated with a layer of palladium oxide/zirconia/alumina using a solution containing the following constituents, per litre of dichloromethane:
25g of palladium acetate
27g of aluminium 2-ethylhexanoate
29.5g of zirconium propionate.

The tile was coated by dip coating in a controlled environment and heated to 120°C at a rate of approximately 40°C per minute in an atmosphere of 4.5% hydrogen in a balance of nitrogen. It was held at 120°C for 2 minutes. This process was repeated 4 times to build up a layer having a suitable thickness to achieve the desired resistance in the final device.

On the 4^{th} cycle, the temperature was ramped up to 350°C at a rate of approximately 100°C per minute and held at this higher temperature for 2 minutes.

It was then exposed to an atmosphere containing 20% oxygen for 5 minutes in order to oxidise the precursors and form the sensor layer.

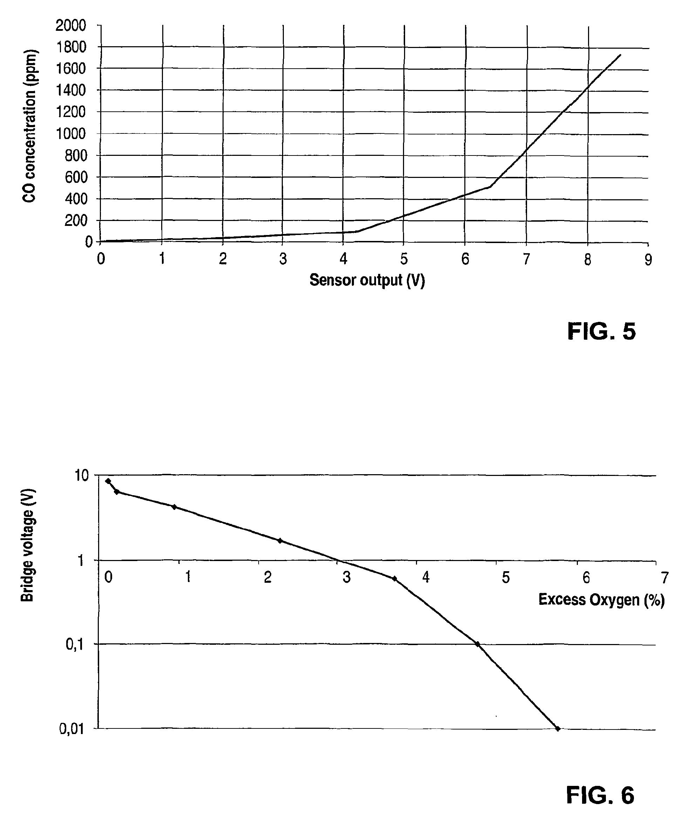

The sensor was tested using a simulated gas boiler comprising a modified Bunsen burner fuelled with natural gas, and having the ability to vary the fuel-air ratio. This allowed a variation in the amount of carbon monoxide (CO) in the exhaust gas from the boiler, which was detected by the sensor, and a response to the corresponding variation in oxygen concentration could then be measured.. The results of the test are shown in Figures 5 and 6.

### EXAMPLE 6

### Preparation of copper coated glass

Firstly, glass was coated with a layer of palladium-containing zirconia/alumina, using a solution containing the following constituents, per litre of terahydrofuran:
80g of zirconium propionate
30g of aluminium 2-ethylhexanoate
20g of palladium acetate

The glass substrate was spin coated with the solution at 2500rpm for 30 seconds, after which it was heated in air at 350°C for 2 minutes then allowed to cool to room temperature.

Thereafter, copper plating was performed using a proprietary electroless plating process supplied by Shipley Europe Ltd. The plating bath is known as Circuposit Electroless Copper 3350, and is composed of four components as follows: 73.8% deionised water, 12.0% Circuposit Electroless Copper 3350M, 4.2% Circuposit Electroless Copper 3350A and 10.0% Circuposit Electroless Copper 3350B, made up in that order. The recommended bath temperature is 46°C, but the samples produced using the present technique have been found to plate very rapidly and for this reason the temperature of the bath was typically 25°C. Continuous air agitation was used during the plating process to stabilise the solution. A layer of copper several hundred nanometres in thickness was produced in a few seconds.

### EXAMPLE 7

### Preparation of patterned nickel coated polyimide

Firstly, a piece of polyimide sheet was coated with a layer of palladium-containing zirconia/alumina, using an identical solution to that described in Example 6. The substrate was spin coated with the solution at 2500rpm for 30 seconds and the solvent was then allowed to evaporate. In order to pattern the ceramic layer, the coated substrate was exposed through a chromium-on-quartz photomask to deep UV light (266nm) for 900s at approximately 20m Wcm⁻² incident power. The substrate was then washed in a 1:1 mixture of acetone and isopropanol, and subsequently rinsed with isopropanol and dried using a nitrogen jet. This removed the unexposed parts of the coating, leaving behind the exposed regions which had been rendered less soluble by the irradiation process.

The patterned substrate was then heated at 350°C for 2 minutes in air after which it was allowed to cool to room temperature. Nickel plating was performed on the sample by immersing it in a proprietary electroless plating solution at a temperature of 90°C for 120s. A layer of nickel of several hundred nanometres thickness was produced only on the remaining regions of palladium-containing zirconia/alumina. Figure 7 shows an example of a patterned nickel layer produced on a polyimide substrate. It was found that this layer of nickel was adhered sufficiently strongly to the substrate that it resisted removal by the Scotch tape test.When this procedure was used with a glass substrate it was found that strongly adherent solder bonds could be made to the nickel.

## Claims

1. A process for the production of a thin film porous ceramic-metal composite, comprising
- contacting a substrate with a solution or solutions comprising precursor compounds of ceramic, stabilising oxide, and metal, so as to form a precursor coating directly on the substrate,
- thermally treating said substrate with the coating at a temperature sufficient to decompose said precursor compounds to form a thin film of stabilised porous ceramic strongly adhered directly to the substrate, the ceramic being in a suitable crystalline form such as zirconia in the cubic phase, incorporating therein or thereon said one or more metals or metal oxides.

2. The process of claim 1, wherein at least one solution is pre-mixed with at least one other solution.

3. The process of claim 1, wherein said substrate comprises metal or metals, or alloys, such as steel containing iron, chromium and aluminium.

4. The process of claim 1, wherein said substrate comprises a knitted wire monolith, such as in the form of a sheet-like material or a roll.

5. The process of claim 1, wherein said substrate comprises silicon, polymers, such as polyimide, and glass.

6. The process of claim 1, wherein said heating is carried out at a temperature in the range from about 350°C to about 1000°C, preferably 400°C for a period between about 10 seconds to about 10 minutes.

7. The process of claim 1, wherein said zirconia precursor is an organic compound of zirconia, such as zirconium substituted or unsubstituted C₁-C₈ alkyl carboxylate, such as propionate.

8. The process of claim 1, wherein said stabilising oxide precursor is an organic compound of yttrium or cerium, such as yttrium substituted or unsubstituted C1-C8 alkyl carboxylate, such as yttrium 2-ethylhexanoate.

9. A composite comprising:
- a thin film porous ceramic layer coated on a substrate, the porous ceramic layer being in a suitable crystalline form such as zirconia in a cubic phase stabilised by a stabilising oxide, wherein the ceramic layer is strongly adhered directly to the substrate, and
- a metal incorporated in or on the ceramic layer.

10. The thin film composite according to claim 9, wherein the substrate is a metal wire, such as a knitted wire material or a knitted wire rolled material.

11. The thin film composite of claim 9, wherein the porous ceramic layer is a zirconia stabilised by yttria.

12. A catalytic element comprising:
- a thin film porous ceramic layer coated on a metal substrate, the porous ceramic layer being in a suitable crystalline form such as zirconia in the cubic phase, stabilised by a stabilising oxide, wherein the ceramic layer is strongly adhered directly to the substrate, and
- a catalytic metal incorporated in or on the ceramic layer.

13. The catalytic element according to claim 12, wherein the substrate is a knitted metallic wire or wires.

14. The catalytic element of claim 12, wherein the catalytic metal is palladium in a concentration from about 0,5% by weight to about 5% by weight to the weight of ceramic layer.

15. A thin film gas sensor comprising:
- a thin film porous ceramic layer coated on a substrate, the porous ceramic layer being in a suitable crystalline form such as zirconia in the cubic phase stabilised by a stabilising oxide, wherein the ceramic layer is strongly adhered directly to the substrate, and
- a metal incorporated in or on the ceramic layer.

16. The thin film gas sensor according to claim 15, wherein the gas sensor has sensitivity with respect to C₁-C₁₈ hydrocarbons, such as propane, butane, etc.

17. A process for the production of a thin film metal plated ceramic-metal composite, comprising the steps of:
- contacting a substrate with a solution to form a coating directly on the substrate, the coating comprising precursors of ceramic, such as zirconia, stabilising oxide, and metal,
- thermally treating said coated substrate at a temperature sufficient to decompose said precursor compounds to form a porous ceramic layer of stabilised ceramic, such as zirconia, adhered directly to the substrate by strong bonding, the ceramic, such as zirconia being in a suitable crystalline form such as the cubic phase incorporating therein or thereon said one or more metals or metal oxides; and
- subjecting the substrate having the ceramic layer thereon to a plating process in conditions to provide incorporated in or on the ceramic layer dispersed metal particles acting as nuclei onto which the metal of the plating process is deposited.

18. A process of claim 17, wherein the ceramic layer is patterned before subjecting to metal plating.

19. A thin film metal plated composite comprising:
- a thin film porous ceramic layer coated on a substrate, wherein the porous ceramic layer, such as zirconia, is adhered directly to the substrate by strong bonding, wherein the ceramic is in a suitable crystalline form such as the cubic phase stabilised by a stabilizing oxide and incorporating one or more metals or metal oxides therein or thereon, and
- a metal film plated upon the ceramic layer.

20. A thin film metal plated composite of claim 19, wherein the metal plated is nickel.

## Patentansprüche

1. Verfahren zur Herstellung eines porösen KeramikMetall-Verbundwerkstoffes in Form eines dünnen Films, umfassend
- das in-Kontakt-bringen eines Substrats mit einer Lösung oder Lösungen, die Vorläuferverbindungen einer Keramik, eines stabilisierenden Oxids, und eines Metalls umfassen, um direkt auf dem Substrat eine Vorläuferbeschichtung zu bilden;
- thermisches Behandeln des Substrats mit der Beschichtung bei einer Temperatur, die ausreichend ist, um die Vorläuferverbindungen zu zersetzen, um einen dünnen Film aus stabilisierter poröser Keramik, die fest direkt an dem Substrat haftet, zu bilden, wobei die Keramik in einer geeigneten kristallinen Form wie zum Beispiel Zirconiumdioxid in der kubischen Phase vorliegt, und das eine oder die mehreren Metalle oder Metalloxide darin oder darauf inkorporiert sind.

2. Verfahren nach Anspruch 1, wobei wenigstens eine Lösung mit wenigstens einer anderen Lösung vorvermischt wird.

3. Verfahren nach Anspruch 1, wobei das Substrat ein Metall oder Metalle oder Legierungen, wie zum Beispiel einen Stahl, der Eisen, Chrom und Aluminium enthält, umfasst .

4. Verfahren nach Anspruch 1, wobei das Substrat ein Monolith aus verknüpftem Draht, wie in der Form eines bogenförmigen Materials oder einer Rolle, umfasst.

5. Verfahren nach Anspruch 1, wobei das Substrat Silicium, Polymere wie zum Beispiel Polyimid, und Glas umfasst.

6. Verfahren nach Anspruch 1, wobei das Erwärmen etwa 10 Sekunden lang bis etwa 10 Minuten lang bei einer Temperatur im Bereich von etwa 350°C bis etwa 1000°C durchgeführt wird, bevorzugt bei einer Temperatur von 400°C.

7. Verfahren nach Anspruch 1, wobei der Zirkoniumdioxid-Vorläufer eine organische Verbindung von Zirkoniumdioxid ist, wie zum Beispiel substituiertes oder unsubstituiertes Zirkonium-C₁-C₈-Alkylcarboxylat, wie zum Beispiel Propionat.

8. Verfahren nach Anspruch 1, wobei der Vorläufer des stabilisierenden Oxids eine organische Verbindung von Yttrium oder Cer ist, wie zum Beispiel substituiertes oder unsubstituiertes Yttrium-C₁-C₈-Alkylcarboxylat, wie zum Beispiel Yttrium-2-Ethylhexanoat.

9. Ein Verbundstoff umfassend:
- eine poröse Keramikschicht in Form eines dünnen Films, die auf ein Substrat beschichtet ist, wobei die poröse Keramikschicht in einer geeigneten kristallinen Form vorliegt, wie zum Beispiel Zirkonium in einer kubischen Phase, stabilisiert durch ein stabilisierendes Oxid, wobei die Keramikschicht direkt an dem Substrat stark haftet, und
ein Metall, das in oder auf der Keramikschicht inkorporiert ist.

10. Der Verbundstoff in Form eines dünnen Films gemäß Anspruch 9, wobei das Substrat ein Metalldraht ist, wie zum Beispiel ein verknüpftes Drahtmaterial oder ein aufgerolltes verknüpftes Drahtmaterial.

11. Der Verbundstoff in Form eines dünnen Films nach Anspruch 9, wobei die poröse Keramikschicht ein durch Yttriumoxid stabilisiertes Zirconiumdioxid ist.

12. Ein katalytisches Element umfassend:
- eine poröse Keramikschicht in Form eines dünnen Films, der auf ein Metallsubstrat beschichtet ist, wobei die poröse Keramikschicht in einer geeigneten kristallinen Form vorliegt, wie zum Beispiel Zirkoniumdioxid in der kubischen Phase, stabilisiert durch ein stabilisierendes Oxid, wobei die Keramikschicht direkt an dem Substrat stark haftet, und
- ein katalytisches Metall, das in oder auf der Keramikschicht inkorporiert ist.

13. Das katalytische Element nach Anspruch 12, wobei das Substrat ein verknüpfter Metalldraht oder -drähte ist.

14. Das katalytische Element nach Anspruch 12, wobei das katalytische Metall Palladium in einer Konzentration von etwa 0,5 Gew.-% bis etwa 5 Gew.-% hinsichtlich des Gewichtes der Keramikschicht ist.

15. Ein Dünnfilmgassensor umfassend:
- eine poröse Keramikschicht in Form eines dünnen Films, die auf ein Substrat beschichtet ist, wobei die poröse Keramikschicht in einer geeigneten kristallinen Form vorliegt, wie zum Beispiel Zirkoniumdioxid in der kubischen Phase, stabilisiert durch ein stabilisierendes Oxid, wobei die Keramikschicht direkt an dem Substrat stark haftet, und
ein Metall, das in oder auf der Keramikschicht inkorporiert ist.

16. Gassensor in Form eines dünnen Films nach Anspruch 15, wobei der Gassensor eine Empfindlichkeit auf C₁-C₁₈ Kohlenwasserstoffe aufweist, wie zum Beispiel auf Propan, Butan, usw.

17. Verfahren zur Herstellung eines metallplattierten Keramik/Metall-Verbundstoffes in Form eines dünnen Films, umfassend die folgenden Schritte:
- in Kontakt-bringen eines Substrats mit einer Lösung zur Bildung einer Beschichtung direkt auf dem Substrat, wobei die Beschichtung Vorläufer einer Keramik wie zum Beispiel Zirconiumdioxid, eines stabilisierenden Oxids, und eines Metalls, umfasst.
- thermisches Behandeln des beschichteten Substrats bei einer Temperatur, die ausreichend ist, um die Vorläuferverbindungen zu ersetzen, um eine poröse Keramikschicht aus stabilisierter Keramik, wie zum Beispiel Zirkoniumdioxid, zu bilden, die durch eine starke Bindung direkt an dem Substrat haftet, wobei die Keramik, wie zum Beispiel Zirconiumdioxid, in einer geeigneten kristallinen Form vorliegt, wie zum Beispiel der kubischen Phase, und darin oder darauf das eine oder die mehreren Metalle oder Metalloxide inkorporiert sind; und
- Unterziehen des Substrats, mit der Keramikschicht darauf, einem Plattierungsverfahren bei Bedingungen, bei denen in oder auf der Keramikschicht inkorporierte dispergierte Metallteilchen bereitgestellt werden, die als Keime agieren, auf denen das Metall des Plattierungsverfahren abgeschieden wird.

18. Verfahren nach Anspruch 17, wobei die Keramikschicht mit einem Muster versehen wird, bevor sie der Metallplattierung unterzogen wird.

19. Ein metallplattierter Verbundstoff in Form eines dünnen Films umfassend:
- eine poröse Keramikschicht in Form eines dünnen Films, die auf ein Substrat beschichtet ist, wobei die poröse Keramikschicht, wie zum Beispiel Zirkoniumdioxid, durch eine starke Bindung direkt auf dem Substrat haftet, wobei die Keramik in einer geeigneten kristallinen Form vorliegt, wie zum Beispiel in Form einer kubischen Phase, die durch ein stabilisierendes Oxid stabilisiert ist, und eines oder mehrere Metalle oder Metalloxide darin oder darauf inkorporiert sind, und
- einen Metallfilm, der auf der Keramikschicht plattiert ist.

20. Ein metallplattierter Verbundstoff in Form eines dünnen Films nach Anspruch 19, wobei das plattierte Metall Nickel ist.

## Revendications

1. Procédé pour la production d'un composite céramique-métal poreux en film mince, comprenant :
- de mettre en contact un substrat avec une solution ou des solutions comprenant des composés précurseurs de céramique, d'oxyde stabilisant et de métal, de façon à former un revêtement précurseur directement sur le substrat,
- de traiter thermiquement ledit substrat avec le revêtement à une température suffisante pour décomposer lesdits composés précurseurs pour former un film mince de céramique poreuse stabilisée fortement collée directement au substrat, la céramique étant sous une forme cristalline appropriée telle que de la zircone en phase cubique, incorporant dans celle-ci ou sur celle-ci lesdits un ou plusieurs métaux ou oxydes de métaux.

2. Procédé de la revendication 1, dans lequel au moins une solution est préalablement mélangée avec au moins une autre solution.

3. Procédé de la revendication 1, dans lequel ledit substrat comprend un métal ou des métaux, ou des alliages, tels que de l'acier contenant du fer, du chrome et de l'aluminium.

4. Procédé de la revendication 1, dans lequel ledit substrat comprend un monolithe de fil métallique tressé, tel que sous la forme d'une matière ressemblant à une feuille ou d'un rouleau.

5. Procédé de la revendication 1, dans lequel ledit substrat comprend du silicium, des polymères, tels que du polyimide, et du verre.

6. Procédé de la revendication 1, dans lequel on effectue ledit chauffage à une température comprise dans l'intervalle allant d'environ 350°C à environ 1000°C, de préférence à 400°C sur une durée comprise entre environ 10 secondes et environ 10 minutes.

7. Procédé de la revendication 1, dans lequel ledit précurseur de zircone est un composé organique de zircone, tel qu'un (alkyle en C₁-C₈ substitué ou non substitué)carboxylate, tel que le propionate, de zirconium.

8. Procédé de la revendication 1, dans lequel ledit précurseur d'oxyde stabilisant est un composé organique de l'yttrium ou du cérium, tel qu'un (alkyle en C₁-C₈ substitué ou non substitué)carboxylate d'yttrium, tel que le 2-éthylhexanoate d'yttrium.

9. Composite comprenant :
- une couche de céramique poreuse en film mince enduite sur un substrat, la couche de céramique poreuse étant sous une forme cristalline appropriée telle que de la zircone en phase cubique stabilisée par un oxyde stabilisant, dans lequel la couche de céramique est fortement collée directement au substrat et
- un métal incorporé dans ou sur la couche de céramique.

10. Composite en film mince selon la revendication 9, dans lequel le substrat est un fil métallique, tel qu'une matière en fil métallique tressé ou une matière enroulée en fil métallique tressé.

11. Composite en film mince de la revendication 9, dans lequel la couche céramique poreuse est une zircone stabilisée par de l'yttria.

12. Elément catalytique comprenant :
- une couche de céramique poreuse en film mince enduite sur un substrat en métal, la couche de céramique poreuse étant sous une forme cristalline appropriée telle que de la zircone en phase cubique, stabilisée par un oxyde stabilisant, dans lequel la couche de céramique est fortement collée directement au substrat, et
- un métal catalytique incorporé dans ou sur la couche de céramique.

13. Elément catalytique selon la revendication 12, dans lequel le substrat est un fil métallique tressé ou des fils métalliques tressés.

14. Elément catalytique de la revendication 12, dans lequel le métal catalytique est le palladium en concentration allant d'environ 0,5 % en poids à environ 5 % en poids par rapport au poids de la couche de céramique.

15. Détecteur de gaz en film mince comprenant :
- une couche de céramique poreuse en film mince enduite sur un substrat, la couche de céramique poreuse étant sous une forme cristalline appropriée telle que de la zircone en phase cubique stabilisée par un oxyde stabilisant, dans lequel la couche de céramique est fortement collée directement au substrat, et
- un métal incorporé dans ou sur la couche de céramique.

16. Détecteur de gaz en film mince selon la revendication 15, dans lequel le détecteur de gaz est sensible aux hydrocarbures en C₁-C₁₈, tels que le propane, le butane, etc.

17. Procédé pour la production d'un composite céramique-métal plaqué de métal en film mince, comprenant les étapes consistant à :
- mettre en contact un substrat avec une solution pour former un revêtement directement sur le substrat, le revêtement comprenant des précurseurs de céramique, telle que de la zircone, d'oxyde stabilisant et de métal,
- traiter thermiquement ledit substrat enduit à une température suffisante pour décomposer lesdits composés précurseurs pour former une couche de céramique poreuse constituée de céramique stabilisée, telle que de la zircone, collée directement au substrat par une liaison forte, la céramique, telle que de la zircone, étant sous une forme cristalline appropriée telle que la phase cubique incorporant dans celle-ci ou sur celle-ci lesdits un ou plusieurs métaux ou oxydes de métaux, et
- soumettre le substrat ayant la couche de céramique sur celui-ci à un procédé de plaquage dans des conditions convenant pour avoir des particules de métal dispersées incorporées dans ou sur la couche de céramique servant de germes sur lesquels le métal du procédé de plaquage est déposé.

18. Procédé de la revendication 17, dans lequel on forme un dessin sur la couche de céramique avant de la soumettre à un plaquage de métal.

19. Composite plaqué de métal en film mince comprenant:
- une couche de céramique poreuse en film mince enduite sur un substrat, dans lequel la couche de céramique poreuse, telle que de la zircone, est directement collée au substrat par une liaison forte, dans lequel la céramique est sous une forme cristalline appropriée telle que la phase cubique stabilisée par un oxyde stabilisant et incorporant un ou plusieurs métaux ou oxydes de métaux dans celle-ci ou sur celle-ci, et
- un film de métal plaqué par-dessus la couche de céramique.

20. Composite plaqué de métal en film mince de la revendication 19, dans lequel le métal plaqué est le nickel.
